# FASCICULE DE BREVET EUROPEEN

(11) **EP 1 804 760 B1**
(45) Date de publication et mention de la délivrance du brevet: **13.08.2008**
(21) Numéro de dépôt: 05805580.7
(22) Date de dépôt: 06.09.2005
(51) Int. Cl.: A61K 8/04

(54) **COMPOSITIONS POUR POILS ET/OU CHEVEUX**
HAARZUSAMMENSETZUNG
HAIR COMPOSITION

(30) Priorité: 07.09.2004 FR 0409440
(43) Date de publication de la demande: 11.07.2007
(73) Titulaire: Merck Chimie SAS, 94120 Fontenay sous Bois (FR)
(72) Inventeur: VIDAL, Richard, F-45000 Orléans (FR)
(74) Mandataire: Ahner, Francis
(86) Numéro de dépôt international: PCT/FR2005/002212
(87) Numéro de publication internationale: WO 2006/027494

(56) Documents cités:
- EP-A- 0 038 730
- EP-A- 1 249 228
- FR-A- 2 825 247
- US-A- 4 339 337

## Description

L'invention concerne des compositions aptes à être appliquées sur les poils ou les cheveux. Spécifiquement, l'invention concerne un mascara, une composition de rasage (mousse, gel, crème ...) ou une composition capillaire (lotion, shampooing ...).

Le mascara est un maquillage très populaire pour les femmes qui souhaitent épaissir ou accentuer leurs cils et l'utilisatrice recherche toujours des mascaras conférant un recourbement important des cils.

Ainsi, le mascara doit permettre une longueur optimale et conférer une couleur, une épaisseur et un recourbement convenable aux cils.

Il est d'usage courant d'utiliser dans les mascaras une proportion variable selon la nature de la formulation de substances filmogènes permettant de conférer une bonne stabilité auxdites compositions.

A l'heure actuelle, les polymères filmogènes utilisés ne permettent pas toujours d'obtenir un recourbement satisfaisant des cils. En outre, certains polymères filmogènes forment sur les cils après séchage, un film blanchâtre conduisant à un maquillage inesthétique.

Ainsi, le brevet EP 0 628 304 décrit-il une composition cosmétique contenant un pseudo-latex qui présente de bonnes propriétés de rémanence, c'est-à-dire qu'elle est difficilement éliminable du support par un simple lavage à l'eau par exemple.

Un besoin existe donc toujours de disposer d'un mascara ayant de bonnes propriétés recourbantes et permettant de former un maquillage invisible.

La coupe ou l'élimination des poils fait typiquement partie de la vie de tous les jours. Que ce soit pour des raisons cosmétiques (rasage du visage, des jambes ...) ou pour des raisons fonctionnelles (préparation d'un malade pour une opération), il existe un besoin de disposer d'une méthode confortable, efficace et peu chère.

La méthode la plus utilisée pour couper ou éliminer les poils met en oeuvre un rasoir, électrique ou non, qui entraîne souvent des irritations de la peau ou des coupures. Bien que l'on utilise des mousses à raser pour réduire le phénomène d'irritation lors du contact de la lame avec la peau, le poil étant coupé simplement au niveau de la peau, le rasage doit être effectué quotidiennement afin de minimiser la repousse du poil, ce qui induit malgré tout des irritations chez les personnes sensibles. L'utilisation d'un rasoir électrique présente les mêmes inconvénients, alors que l'utilisation de la cire, si elle évite un usage quotidien, est souvent très douloureuse.

Aussi, un besoin existe toujours de disposer d'une mousse à raser qui permette un rasage efficace et moins douloureux.

Le brevet US 6 463 661 décrit une méthode de rasage mettant en oeuvre un rasoir muni d'une lame couplée à un aimant et comprenant l'application, avant rasage, d'une solution contenant des particules magnétiques, sur les poils, de manière à les recouvrir desdites particules magnétiques, et l'utilisation du rasoir de manière à ce que l'aimant applique sa force magnétique sur lesdites particules magnétiques, conduisant ainsi au redressement des poils pour faciliter le rasage. Les particules magnétiques utilisées sont des particules métalliques, notamment à base de Fe₃O₄ obtenu à partir d'un sable. Toutefois, ces particules sont utilisées directement sans aucune purification et aucune formulation précise n'est décrite.

La présente invention a donc pour but de proposer des compositions aptes à être appliquées sur les poils ou les cheveux pour les redresser. Dans le cas de la composition de mascara, la composition présente de bonnes propriétés recourbantes des cils et une homogénéité d'application qui leur confère un aspect naturel.

La présente invention a en conséquence pour objet des compositions aptes à être appliquées sur les poils ou les cheveux, comprenant une suspension aqueuse de microsphères de latex magnétique et de nanoparticules colloïdales de pigment magnétique.

Dans un mode de réalisation préféré de l'invention, la composition apte à être appliquée sur les poils ou les cheveux est un mascara, une composition de rasage ou une composition capillaire comprenant une suspension aqueuse de microsphères de latex magnétiques et de nanoparticules colloïdales de pigment magnétique.

On entend au sens de mascara, les éléments basiques pour la formulation de mascara, notamment un mascara comprenant un milieu cosmétiquement acceptable, c'est-à-dire un milieu compatible avec les matières kératiniques.

Au sens de la présente invention, on entend par latex magnétique les latex tels que ceux décrits dans le brevet européen EP 0 038 730 et le brevet américain US 4 339 337 et par nanoparticules colloïdales de pigment magnétique des nanoparticules constituées par un mélange d'oxyde de Fe₃O₄ enrobés par un tensioactif ionique ou non ionique tels que ceux décrits dans le brevet européen EP 0 038 730 et le brevet américain US 4 339 337.

Dans un mode de réalisation préféré de l'invention, le latex magnétique représente de 5 à 20% du poids total de la composition et le pigment magnétique représente 15 à 30% en poids de la composition.

Dans un autre mode préféré de l'invention, le diamètre des microsphères de latex magnétiques est compris entre 100 nanomètres et 2 micromètres, de préférence entre 700 et 1,3 micromètre et le diamètre des nanoparticules colloïdales de pigment magnétique est compris entre 2 et 50 nanomètres, de préférence entre 5 et 20 nanomètres.

Les lotions capillaires et les formulations de rasage, auxquelles on ajoute les microsphères de latex magnétiques et les nanoparticules colloïdales de pigment magnétique, peuvent être n'importe quel type de solutions de compositions connues de l'homme du métier, telles que les crèmes, les gels, les mousses et les lotions.

Les compositions de mascara selon l'invention peuvent être également n'importe quelles compositions de mascara connues de l'homme du métier.

Elles peuvent contenir des composés antistatiques. On peut citer à titre d'exemple d'antistatiques, les agents tensioactifs anioniques, non ioniques, cationiques, amphothériques, les polysaccharides tels que la chitine ou ses dérivés, le chitosan et ses dérivés.

Les compositions de mascara selon l'invention peuvent également contenir des agents filmogènes qui confèrent une bonne résistance au mascara. Parmi les agents filmogènes cosmétiquement acceptables, on peut citer par exemple les polymères comme les polymères acryliques, les polymères à base de polyéthylène, les copolymères de polyvinylpyrrolidone, les acétates d'éthylène de vinyle, la gomme de diméthicone et les résines, comme les résines silicones.

Elles peuvent contenir éventuellement d'autres additifs usuels, par exemple des anti-oxydants. Parmi les anti-oxydants appropriés, on peut citer le propylparabène, le butylparabène ou leurs mélanges.

Les mascaras peuvent également contenir un ou plusieurs pigment(s) approprié(s) pour l'utilisation autour des yeux. Comme exemple de pigments, on peut citer les oxydes métalliques, l'oxychlorure de bismuth et l'oxyde de chrome. On peut également ajouter des agents viscosifiants tels que les cires et autre gélifiants, ainsi que des charges telles que le nylon, l'albumine, le talc et autres charges typiquement utilisées dans ces compositions.

Les mascaras selon l'invention peuvent se présenter sous la forme de gel aqueux, de dispersion cire/eau, eau/cire, huile/eau et eau/huile.

L'homme du métier veillera à choisir ces éventuels composés complémentaires et/ou leurs quantités, de manière à ce que les propriétés avantageuses de la composition selon l'invention ne soient pas altérées par l'adjonction envisagée.

Les compositions selon l'invention peuvent être préparées selon les méthodes usuelles des domaines considérés.

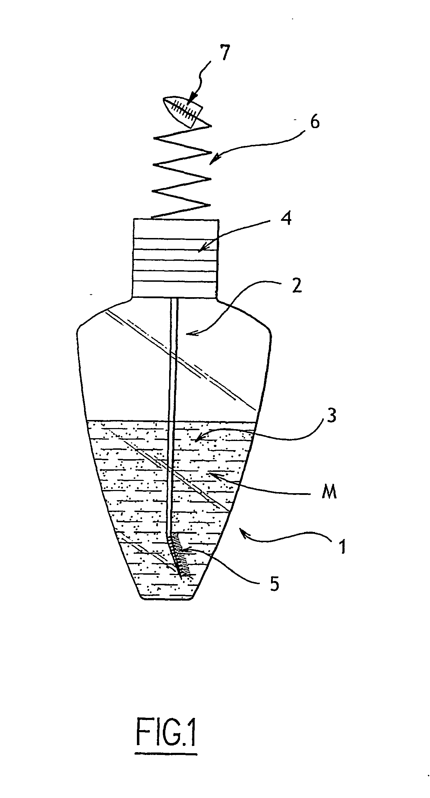

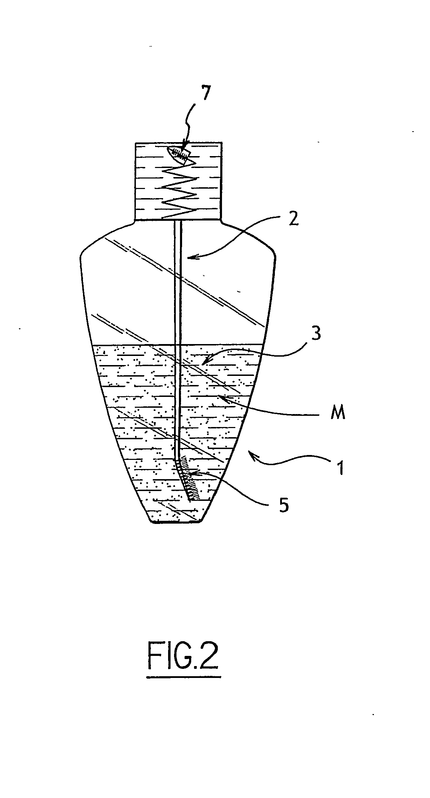

L'invention a également pour objet un dispositif pour le conditionnement et l'application d'un mascara illustrés aux figures 1 à 3 et comportant un récipient (1) contenant ledit mascara (M) à appliquer et un applicateur (2). L'applicateur (2) comprend une tige (3) munie à une extrémité d'un organe de préhension (4) qui constitue le bouchon de fermeture du récipient (1) et, à l'autre extrémité, d'une brosse (5) constituée de fibres destinée à l'application du mascara.

Au sein de l'organe de préhension (4), dont la longueur est de préférence d'environ de 2/3 de celle du récipient (1), est incorporée une tige dépliable (6), dont l'extrémité est constituée d'une brosse à poils ras (7) susceptible d'induire un champ magnétique compris entre 0,7 et 1,5 tesla.

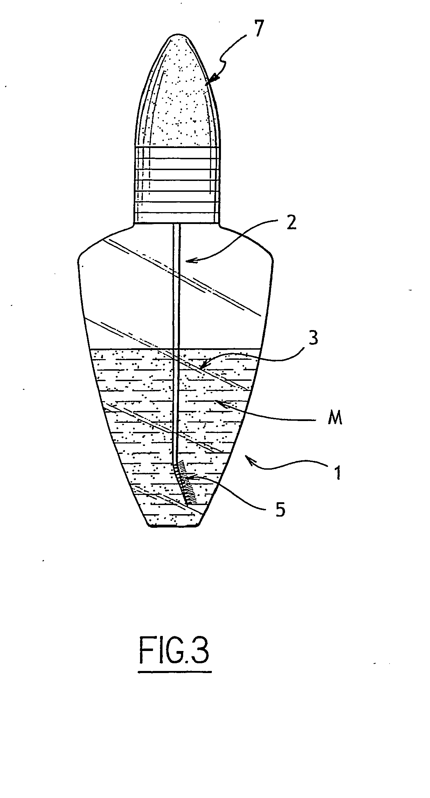

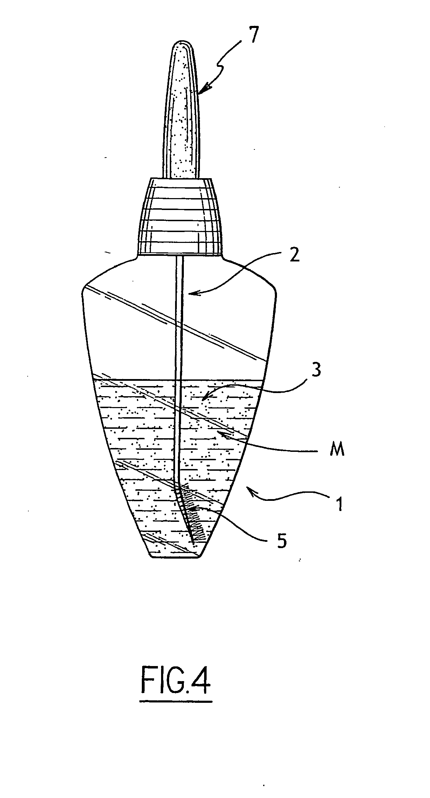

Dans d'autres modes de réalisation du dispositif illustrés dans les figures 3 et 4, l'organe de préhension (7) peut être lui-même un aimant.

Les compositions de rasage selon l'invention peuvent être utilisées avec n'importe quel appareil de rasage comportant un aimant à proximité de la lame, tel celui décrit dans les brevets US 6 327 779, US 2001/0022025 et US 6 463 661.

L'effet de la présence du latex magnétique et des nanoparticules magnétiques dans les compositions de rasage, lorsque celles-ci sont appuyées sur le poil, permettent au poil de se redresser au-dessus de la peau et d'obtenir un meilleur rasage, tout en diminuant le contact abrasif de la lame avec la peau.

Le champ magnétique peut être créé par l'intermédiaire d'un aimant ferrique, par exemple un aimant néodyme/fer/bore, ou samarium/colbat.

L'invention concerne également l'utilisation d'une composition selon l'invention pour redresser les poils.

Plus particulièrement, l'invention concerne l'utilisation d'un mascara contenant une suspension aqueuse de microsphères de latex magnétiques pour recourber les cils.

L'invention a également pour objet l'utilisation d'une suspension aqueuse de microsphères de latex magnétiques et de nanoparticules colloïdales de pigment magnétique dans une composition de mascara comprenant un milieu cosmétiquement acceptable pour recourber les cils.

L'invention a encore pour objet un procédé de revêtement des cils comprenant l'application sur les cils d'une composition de mascara telle que définie précédemment .

Dans un mode préféré de réalisation de l'invention, le procédé de revêtement des cils est mis en oeuvre avec un élément applicateur tel que décrit précédemment.

Le revêtement des cils est en particulier un procédé de maquillage de traitement cosmétique non thérapeutique des cils.

La composition de mascara selon l'invention est facilement utilisée par le consommateur. Elle est simplement appliquée sur les cils supérieurs ou inférieurs et sèche en 1 minute ou 2. Les cils sont longs, bien séparés et ont une apparence naturelle.

Les compositions magnétiques appliquées sur les poils ou les cheveux, notamment sur les cils, les rendent magnétisables et permettent donc, lorsqu'ils sont soumis à des champs magnétiques, de les orienter dans une direction voulue. La force magnétique appliquée au mascara magnétique procure en outre l'énergie nécessaire pour franchir les barrières énergétiques réactionnelles et aboutir ainsi à l'obtention d'un produit stable sur les cils, les maintenant pendant plusieurs heures dans la direction du champ magnétique après son action.

L'exemple et les figures qui suivent illustrent plus en détail l'invention:
La **figure 1** représente un dispositif pour le conditionnement et l'application de mascara selon l'invention prêt à être utilisé.
La **figure 2** illustre un dispositif pour le conditionnement et l'application du mascara selon l'invention à l'état de conditionnement.
Les **figures 3** **et** **4** illustrent un dispositif dans lesquels l'organe de préhension est lui-même un aimant.

### Exemple: Formulation de mascara

Il est préparé selon des techniques connues de l'homme du métier par mélange de polymères filmogènes solubles dans l'eau et de nanosphères polymériques en dispersion dans l'eau.

**Copolymères d'acétate de vinyle et de vinylpyrrolidone**

| | |
|---|---|
| | 23% |
| Eau | 30% |
| Huile de silicone | 5% |
| Triéthanolamine | 2% |
| Microsphères de latex magnétique | 5-20% |
| Nanoparticules de pigment magnétique | 15-30% |

## Revendications

1. Compositions aptes à être appliquées sur les poils ou les cheveux, comprenant une suspension aqueuse de microsphères de latex magnétique et de nanoparticules colloïdales de pigment magnétique.

2. Compositions selon la revendication 1 qui est choisie dans le groupe comprenant un mascara, une composition de rasage et une composition capillaire.

3. Compositions selon l'une quelconque des revendications 1 et 2 dans lesquelles le latex magnétique représente de 5 à 20% du poids total de la composition et le pigment magnétique représente 15 à 30% en poids de la composition.

4. Compositions selon l'une quelconque des revendications 1 à 3 dans lesquelles le diamètre des microsphères de latex magnétique est compris entre 10 nanomètres et 2 micromètres, de préférence entre 700 et 1,3 micromètre et le diamètre des nanoparticules colloïdales de pigment magnétique est compris entre 2 et 50 nanomètres, de préférence entre 5 et 20 nanomètres.

5. Dispositif pour le conditionnement et l'application d'un mascara selon l'une quelconque des revendications précédentes et comportant un récipient (1) contenant ledit mascara à appliquer et un applicateur (2), ledit applicateur (2) comprenant une tige (3) munie à une extrémité d'un organe de préhension (4) qui constitue le bouchon de fermeture du récipient (1) et, à l'autre extrémité, d'une brosse (5) constituée de fibres destinée à l'application du mascara, **caractérisé en ce que**, au sein de l'organe de préhension (4) est incorporée une tige dépliable (6) dont l'extrémité est constituée d'une brosse à poils ras (7) susceptible d'induire un champ magnétique compris entre 0,7 et 1,5 tesla.

6. Utilisation d'une composition selon l'une quelconque des revendications 1 à 4 pour redresser les poils.

7. Utilisation d'une suspension aqueuse de microsphères de latex magnétiques et de nanoparticules colloïdales de pigment magnétique dans une composition de mascara comprenant un milieu cosmétiquement acceptable pour recourber les cils.

8. Procédé de revêtement des cils comprenant l'application sur les cils d'une composition de mascara selon l'une quelconque des revendications 1 à 4.

## Claims

1. Compositions which can be applied to body hairs or head hair, comprising an aqueous suspension of magnetic latex microspheres and of colloidal nanoparticles of magnetic pigment.

2. Compositions according to Claim 1, which are chosen from the group comprising a mascara, a shaving composition and a hair composition.

3. Compositions according to one of Claims 1 and 2, in which the magnetic latex represents from 5 to 20% of the total weight of the composition and the magnetic pigment represents 15 to 30% by weight of the composition.

4. Compositions according to any one of Claims 1 to 3, in which the diameter of the magnetic latex microspheres is between 10 nanometres and 2 micrometres, preferably between 700 and 1.3 micrometres, and the diameter of the colloidal nanoparticles of magnetic pigment is between 2 and 50 nanometres, preferably between 5 and 20 nanometres.

5. Device for the conditioning and application of a mascara according to any one of the preceding claims and comprising a container (1) containing said mascara to be applied and an applicator (2), said applicator (2) comprising a stem (3) equipped at one end with a gripping member (4) which constitutes the cap for closing the container (1) and, at the other end, a brush (5) consisting of fibres intended for application of the mascara, **characterized in that**, incorporated within the gripping member (4) is a stem which can be opened out (6), the end of which consists of a short-haired brush (7), capable of inducing a magnetic field between 0.7 and 1.5 tesla.

6. Use of a composition according to any one of Claims 1 to 4, for making body hairs stand up.

7. Use of an aqueous suspension of magnetic latex microspheres and of colloidal nanoparticles of magnetic pigment in a mascara composition comprising a cosmetically acceptable medium, for curling the eyelashes.

8. Method for coating the eyelashes, comprising the application onto the eyelashes of a mascara composition according to any one of Claims 1 to 4.

## Patentansprüche

1. Zusammensetzungen, die dazu geeignet sind, auf die Körper- oder Kopfhaare aufgetragen zu werden, umfassend eine wässrige Suspension magnetischer Latex-Mikrokugeln und kolloidaler Nanopartikel magnetischen Pigments.

2. Zusammensetzungen nach Anspruch 1, die in der Gruppe mit einer Wimperntusche, einer Rasurzusammensetzung und einer Haarzusammensetzung gewählt werden.

3. Zusammensetzungen nach einem der Ansprüche 1 und 2, in denen das magnetische Latex 5 bis 20 % des Gesamtgewichts der Zusammensetzung umfasst und das magnetische Pigment 15 bis 30 % des Gewichts von der Zusammensetzung umfasst.

4. Zusammensetzungen nach einem der Ansprüche 1 und 3, in denen der Durchmesser der magnetischen Latex-Mikrokugeln zwischen 10 Nanometern und 2 Mikrometern ist, vorzugsweise zwischen 700 und 1,3 Mikrometer und in denen der Durchmesser der kolloidalen Nanopartikel magnetischen Pigments zwischen 2 und 50 Nanometer, vorzugsweise zwischen 5 und 20 Nanometer ist.

5. Vorrichtung zum Verpacken und zum Auftragen einer Wimperntusche nach einem der vorhergehenden Ansprüche und umfassend ein Gefäß (1) mit der aufzutragenden Wimperntusche und mit einem Applikator (2), wobei der Applikator (2) einen Stab (3) umfasst, der an einem Ende mit einem Greiforgan (4) versehen ist, das den Verschlussstopfen des Gefäßes (1) bildet und, der am anderen Ende mit einer Bürste (5) versehen ist, die sich aus Fasern zusammensetzt, die zum Auftragen der Wimperntusche vorgesehen sind, **dadurch gekennzeichnet**, das im Inneren des Greiforgans (4) ein entfaltbarer Stab (6) untergebracht ist, dessen Ende sich aus einer Bürste mit kurzen Haaren (7) zusammensetzt, die ein magnetisches Feld zwischen 0,7 und 1,5 Tesla induzieren kann.

6. Verwendung einer Zusammensetzung nach einem der Ansprüche 1 bis 4, um die Haare aufzurichten.

7. Verwendung einer wässrigen Suspension magnetischer Latex-Mikrokugeln und kolloidaler Nanopartikel magnetischen Pigments in einer Wimperntuschenzusammensetzung umfassend ein kosmetisch annehmbares Milieu, um die Wimpern nach oben zu biegen.

8. Beschichtungsverfahren der Wimpern umfassend das Auftragen einer Wimperntuschenzusammensetzung nach einem der Ansprüche 1 bis 4 auf die Wimpern.
